# EUROPEAN PATENT APPLICATION

(11) **EP 4 043 587 A1**
(43) Date of publication of application: **17.08.2022**
(21) Application number: 21158255.6
(22) Date of filing: 19.02.2021
(51) Int. Cl.: C12Q 1/6883

(54) **DIAGNOSTIC MICRO-RNA KIT FOR THE EVALUATION OF THE INFLAMMATORY ACTIVITY OF PERIODONTITIS IN PATIENTS WITH RHEUMATOID ARTHRITIS**

(30) Priority: 11.02.2021 LT 2021004
(71) Applicant: Vilnius University Hospital Zalgiris Clinic, 08217 Vilnius (LT)
(72) Inventor: Puriene, Alina, 03104 Vilnius (LT); Jarmalaite, Sonata, Vilnius (LT); Butrimiene, Irena, 01124 Vilnius (LT); Punceviciene, Egle, 02222 Vilnius (LT); Snipaitiene, Kristina, 03101 Vilnius (LT); Rovas, Adomas, 06327 Vilnius (LT)
(74) Representative: Petniunaite, Jurga

(57) **Abstract**

The present invention refers to an application of microRNAs (miRNAs), particularly miR-140-3p, -145-5p, -146a-5p and -195-5p, for the diagnosis of periodontitis (PD). The present invention provides a method and a kit for diagnosing inflammatory activity of periodontitis in biological fluid samples, including gingival crevicular fluid (GCF), saliva and plasma, of patients with and without rheumatoid arthritis. Encompassed are methodological steps and materials needed for sample collection, RNA extraction, targeted complementary DNA synthesis, reverse transcription quantitative PCR and data analysis to determine the relative level of inflammatory PD-specific miRNAs, particularly miR-140-3p, -145-5p, -146a-5p and -195-5p. The results show that PD can be sensitively and specifically diagnosed by the analysis of above mentioned miRNAs.

## Description

### FIELD OF THE INVENTION

The invention relates to the field of medical technology, the medical use of a known substance - micro-RNA (miRNA) for the diagnosis of periodontitis (PD) inflammatory activity in patients with and without rheumatoid arthritis (RA) and other systemic diseases. It is based on a modified miRNA detection, isolation, and identification methodology in bodily fluids (gingival crevicular fluid (GCF), saliva and plasma).

### BACKGROUND OF THE INVENTION

PD is a chronic infectious disease characterized by inflammatory destruction of the connective tissue, resulting in loss of the periodontal junction and resorption of the alveolar bone. PD is the most common chronic inflammatory disease, with a frequency of 10 up to 70 percent in the adult population, depending on the age group and diagnostic criteria used in the studies (1). The prevalence of the disease increases in proportion to the age of the population - it was found that 70% of the population over 65 years have PD (2). Given the aging trend of the population and the increasing incidence of risk factors for PD (obesity, diabetes, etc.), a higher prevalence of PD is predicted in the future (3). The extremely high prevalence of periodontal disease is associated with the asymptomatic course of the disease, where individuals seek treatment only when there are late signs of the disease - abscess in the gums, tooth mobility, tooth loss, and so on. Despite the fact that PD is the main cause of tooth loss, the negative impact of periodontal pathology is not limited to changes in the mouth. Prolonged, chronic increase in anaerobic bacteria in the mouth is associated with an increased risk of bacterial pneumonia, chronic obstructive pulmonary disease, and other respiratory diseases and cancers (4). The entry of periodontal pathogenic bacteria into the bloodstream during daily procedures (brushing, flossing, chewing, etc.) contributes to the formation of atherosclerotic lesions, increases the risk of developing infectious endocarditis and infarction (5, 6). However, PD causes the greatest adverse effects to the body due to the low intensity of chronic inflammation, which activates the immune system and leads to increased production of inflammatory mediators (7). Increased production of tumour necrosis factor α (TNF-α), interleukin-6 (IL-6), and other inflammatory cytokines is a major pathogenetic mechanism linking PD and autoimmune diseases (8). The autoimmune inflammatory disease most commonly associated with PD is RA. The incidence of PD in patients with RA is up to twice as high as in the general population and can affect as much as 97% of patients with RA (9). Moreover, the clinical course of periodontal pathology correlates with RA disease activity, and a decrease in the clinical activity of rheumatoid arthritis is observed after successful periodontal treatment (10). In PD and RA, increased production of inflammatory cytokines is detected, which leads to resorption of connective and bone tissue (8). These diseases are also associated with similar epigenetic regulation: methylation of the IL-6 gene promoter is lower in patients with RA and PD compared to healthy subjects, resulting in elevated serum IL-6 levels (11).

It is known that inflammation in periodontal tissues is initiated by the accumulation of microorganisms on the tooth surface. The by-products of these microorganisms activate the human immune system, causing the infiltration of lymphocytes, macrophages and polymorphonuclear cells into the inflammatory site. Bacterial lipopolysaccharide-induced macrophages secrete large amounts of inflammatory cytokines: interleukin-1 (IL-1), TNF-α, prostaglandin E2 (PGE2), and so on. Meanwhile, T lymphocytes activated by pathogenic bacteria produce IL-1 and lymphotoxin. Inflammatory cytokines mediate catabolic processes in the focal periodontal lesion by stimulating the action of metalloproteinases and other enzymes capable of degrading collagen (12). Both direct bacterial activity and the immune cell response contribute to periodontal tissue destruction and disease progression (13). Despite the significant influence of microorganisms in the early stages of PD, it is estimated that the accumulation of microorganisms in periodontal tissues is responsible for only 20% increase in the risk of advanced periodontal disease (14). The rate of disease progression and the overall adverse effects on the body depend on the intensity of the inflammatory response.

Inflammation is a complex pathophysiological mechanism regulated by the interactions of various mediators. Pathogen recognition receptors normally activated in immune cell membranes during inflammation induce activation of nuclear factor kappa B (NF-κB) (15). NF-κB initiates transcription of target genes by binding to specific DNA sequences in the nucleus of the cell (16). As a result, the production of inflammatory cytokines, chemokines, anti-apoptotic factors, and adhesion molecules in the cell is stimulated (16). Increased concentrations of major inflammatory cytokines (TNF-α, IL-1β, IL-6) lead to differentiation and migration of immune cells to the site of inflammation and further progression of inflammation. Each stage of the inflammatory cascade is regulated by negative and positive feedback. Molecules involved in the transcription of genes encoding inflammatory mediators in the nucleus or in the cytoplasm have a particularly high regulatory potential. For example, histone acetylation induced by acetyl coenzyme A (Acetyl-CoA) results in activated transcription of the interferon-gamma (IFNγ) gene in the nucleus, and IFNγ promotes immune T cell differentiation (17). Meanwhile, the expression of genes transcribed in the nucleus may be regulated by another epigenetic factor, miRNA (20). These are small (average 22 nucleotides in length), single-stranded, non-coding RNA molecules that mainly negatively affect gene expression by binding to reference RNA (mRNA) and, depending on the level of sequence matching, initiating transcript degradation or inhibiting translation in ribosomes and thereby inhibiting specific synthesis (20). Various miRNAs are involved in the regulation of the inflammatory response. According to a 2018 study by the Fushimi group of researchers, miR-140-3p is involved in osteoblast differentiation processes by mediating between Wnt3 and TGFβ3 signalling pathways, but a more detailed mechanism is not yet known (19). In addition, Taguchi and co-authors single out this miRNA as one of the most potential biomarkers for the diagnosis of PD (20). Meanwhile, miR-145 inhibits IL-6 and IL-8 expression and reduces IL-1β-induced membrane degradation (21, 22). In an experimental study in mice, inhibition of miR-145 resulted in increased macrophage infiltration and inflammation in the liver, and conversely, experimental increase in miR-145 expression inhibited macrophage differentiation, infiltration, and overall inflammatory intensity (23). MiR-146a is also significantly involved in the regulation of inflammation (24). Insufficient expression of miR-146a has been associated with inflammatory and autoimmune diseases such as RA, systemic lupus erythematosus, and psoriasis (25). NF-κB promotes an increase in miR-146a expression, which in turn binds to pathogen-recognizing membrane receptors and inhibits further activation of NF-κB (26). With such negative feedback, miR-146a inhibits further progression of the inflammatory response. In addition, miR-146a reduces the production of IL-1β, IL-6, IL-8, and TNF-α, the formation of active oxygen forms, and macrophage chemotaxis, and thus contributes to the suppression of the hyperergic inflammatory response (27, 28). Because of these properties, miR-146a is considered to be one of the most promising molecules for the diagnosis and treatment of various inflammatory diseases.

It is clear that the intensity of the inflammatory response is a key diagnostic and prognostic indicator in assessing the course of PD, the risk of further progression, and adverse systemic effects on the health of the body as a whole. However, the clinical methods currently widely used in periodontal practice - complete periodontal examination and radiological examination - are based on the assessment of the severity of tissue destruction that has already occurred due to periodontal disease, and not on the determination of inflammatory activity. The only clinical parameter used to assess disease intensity is the post-probing bleeding index. This index does not always objectively reflect the course of the disease, as the accuracy of the index depends on many factors: the clinician's probing skills, the force used for probing, the medication used by the patient, and so on. It is worth noting that in a smoker patient, the application of this index is not rational due to changes in the blood vessels of the gums. The obvious need for new diagnostic markers and new diagnostic methods for the assessment of PD is emphasized in 2018 World Congress report of the European Federation of Periodontology (EFP) and the American Academy of Periodontology (AAP) (29).

Saliva is one of the bodily fluids and is considered the most appropriate biological medium for the non-invasive diagnosis of PD and other diseases for several reasons (30). First of all, saliva collection does not cause discomfort to the patient and can be performed not only in a specialized treatment facility, but also in primary care facilities, which increases the likelihood of early detection of the disease - before the onset of late symptoms. In addition, a variety of locally excreted substances of organic origin, including proteins, metabolites, and genetic material, are found in saliva. A team of researchers led by Kooijman uses the identification of protein combinations in saliva to detect mild and advanced PD (EP 3553525). The combination of protein pyruvate kinase (PK), matrix metalloproteinase-9 (MMP-9), S100 calcium binding protein A9 (S100A9), S100 calcium binding protein A8 (S100A8), hemoglobin-forming chains β (Hb-beta) is quantified and qualitatively determined. Also, in order to distinguish mild PD from advanced, Chatterjea recommends the qualitative and quantitative identification of the combination of IL-1β, IL-6, MMP-9, MMP-3 proteins found in saliva (EP 3407068). In order to improve the accuracy of the diagnostic kit, Bakker et al. recommends that the assessment of MMP-8 and hepatocyte growth factor (HGF) levels be combined with demographics (gender, body mass index) (EP 3407070).

The already mentioned potential of miRNA to regulate the homeostasis of various body processes and extremely favourable diagnostic properties (small molecular size, simple and accurate identification, etc.) have made miRNA one of the most widely studied and diagnostically applicable genetic materials. There are currently 5 commercial diagnostic kits that have successfully passed the clinical trial stages (31). "ThyraMIR" (*Interpace Diagnostics)* is a diagnostic kit based on the identification of 10 miRNAs to detect the malignancy of thyroid cysts and to assess the need for surgical intervention. Since 2018, testing with "ThyraMIR" in the United States is state-funded and is recommended prior to surgical removal of thyroid cysts. Meanwhile, "miRview mets" (*Oncotest*) is used to identify miRNAs of malignant metastases to determine the origin of the tumour. *TAmiRNA,* the European leader in miRNA diagnostics, offers the identification of circulating miRNA combinations for the assessment of osteoporosis ("OsteomiR"), platelet function ("ThrombomiR") and tissue toxic damage ("ToxomiR"). Other diagnostic kits for the evaluation of cancers, Alzheimer's disease, and hepatic toxin damage are in the phases of clinical trials (31).

The aforementioned patents applications for PD diagnostic kits (EP 3553525; EP 3407068; EP 3407070) were filed before July 2018, when the new classification of periodontal diseases was approved by EFP and PPE was published (29). Starting from 2018, the current classification of periodontal diseases presents significant changes, based on which the diagnosis of the disease is made not on the basis of the amount of lost periodontal tissue (bone, gums, periodontal joint), but on the basis of current periodontal tissue health. An established diagnosis is a corresponding periodontal condition with a previous loss of periodontal connection (29). According to the 1999 - 2018 classification, this diagnosis would correspond to a moderate or severe PD (32). Diagnostic methods based on the previous classification of periodontal diseases are not compatible with the currently used disease classification criteria and do not reflect disease activity, which may result in incomplete accuracy. Also, despite the fact that the selected combinations of proteins or miRNAs are associated with inflammatory processes, the diagnostic kits themselves are based on an assessment of the PD disease rather than the inflammatory activity. Importantly, the effects of systemic anti-inflammatory and immunosuppressive drugs have not been documented in assessing the accuracy of these diagnostic kits. It is known that these drugs can mask the severity of chronic PD by reducing the amount of inflammatory mediators in the body (9). For this reason, the specified diagnostic kits should not be used for the diagnosis of PD in patients taking systemic anti-inflammatory and immunosuppressive agents (e.g., for RA or other autoimmune diseases).

### SUMMARY OF THE INVENTION

The diagnostic kit we developed is based on the identification of inflammatory-related miRNAs in bodily fluids and is therefore less invasive. This method is unique in that it allows to identify the inflammatory activity of PD in both rheumatologically healthy and patients with a disease pathogenetically closely related to PD - rheumatoid arthritis. Our methodology for the determination of PD-related inflammatory activity consists of several main steps: collection of liquid biopsy samples, isolation of RNA using a miRNeasy Mini Kit (*Qiagen*) based on silica gel columns, and quantitative reverse transcription polymerase chain reaction (RT-qPCR) analysis of miRNA in liquid biopsy samples. The present invention provides a minimally- and non-invasive diagnostic method based on the identification of GCF, salivary and plasma miRNAs for assessing the inflammatory activity of PD in patients with PD and/or rheumatoid arthritis. The invention enables a minimally- and non-invasive diagnosis of PD and is also used to identify PD activity that is relevant to the choice of further periodontal treatment tactics. The invention has been applied to patients with rheumatoid arthritis in order to quickly and accurately assess the intensity of inflammation caused by PD. The need for this diagnostic approach for the target population is based on the fact that active inflammation of any origin in RA patients adversely affects the clinical course of RA and may limit the use of drugs to modify the course of RA (e.g., methotrexate, biologics, etc.).

### BRIEF DESCRIPTION OF FIGURES

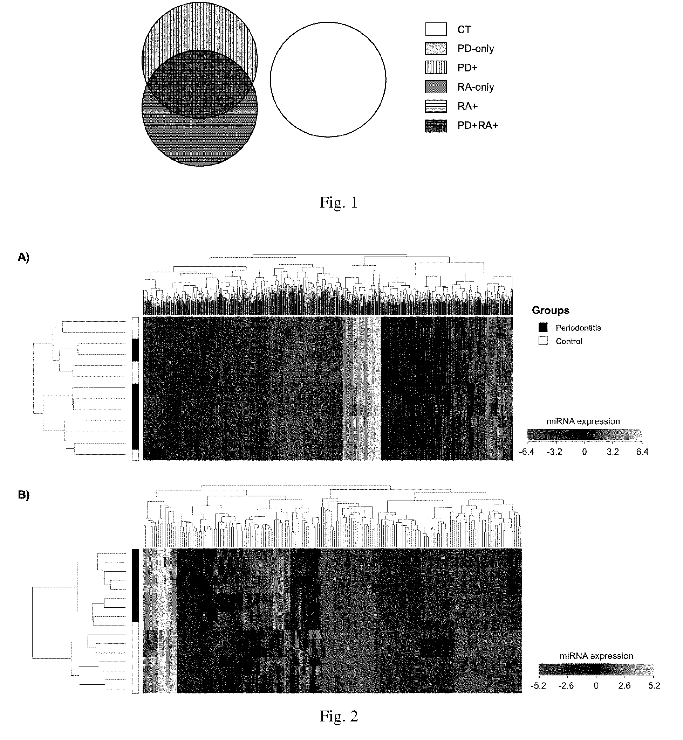

**Figure 1**. Participant groups according to the status of periodontitis (PD) and rheumatoid arthritis (RA). CT - healthy controls (PD-RA-), PD-only - patients with PD (PD+RA-), PD+ - patients with PD, regardless the status of RA, RA-only - patients with RA (RA+PD-), RA+ - patients with RA, regardless the status of PD, and PD+RA+ - patients with both PD and RA.
**Figure 2**. Hierarchical clustering of differentially expressed miRNAs in periodontitis and control gingival tissue samples: miRNAs that were detected in ≥ 25% of the samples (A) and significantly deregulated miRNAs (N = 177, FC ≥ 1.5, P ≤ 0.050) (B).

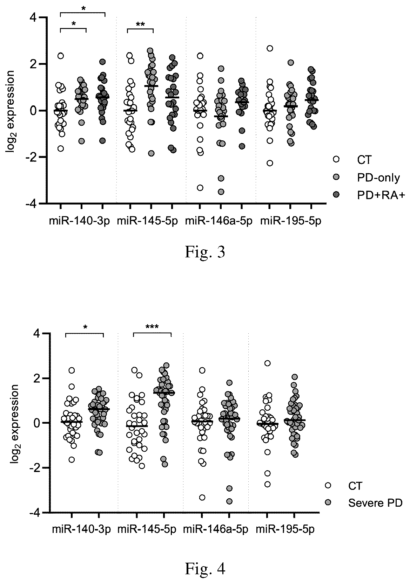

**Figure 3****.** Relative expression of miR-140-3p, -145-5p, -146a-5p, and -195-5p in gingival tissues evaluated by means of RT-qPCR. Comparison of patients with periodontitis (PD-only), patients with both PD and RA (PD+RA+) and healthy controls (CT). The black lines denote median values, * P < 0.050, ** P < 0.010.
**Figure 4****.** Relative expression of miR-140-3p, -145-5p, -146a-5p, and -195-5p in gingival tissues collected from patients with severe periodontitis (PD, stage III and IV) and healthy controls (CT). The black lines denote median values, * P < 0.050, *** P < 0.001.

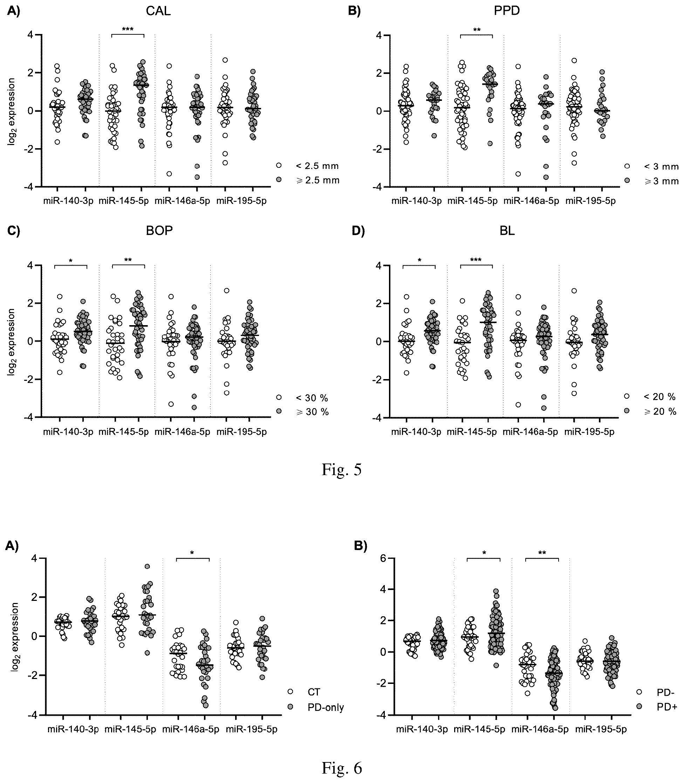

**Figure 5****.** Relative expression of miR-140-3p, -145-5p, -146a-5p, and -195-5p in gingival tissues collected from periodontitis patients and controls and compared according to cut-off points of mean clinical attachment loss (CAL) (A), periodontal probing depth (PPD) (B), bleeding on probing (BOP) (C) and bone loss (BL) (D). The black lines denote median values, * P < 0.050, ** P < 0.010 and *** P < 0.001.
**Figure 6****.** Comparison of miR-140-3p, -145-5p, -146a-5p, and -195-5p level in gingival crevicular fluid collected from patients with periodontitis (PD-only) *vs.* healthy controls (CT) (A), and all patients with PD (PD+) vs. periodontally healthy patients (PD-), regardless the status of rheumatoid arthritis (B). The black lines denote median values, * P < 0.050 and ** P < 0.010.

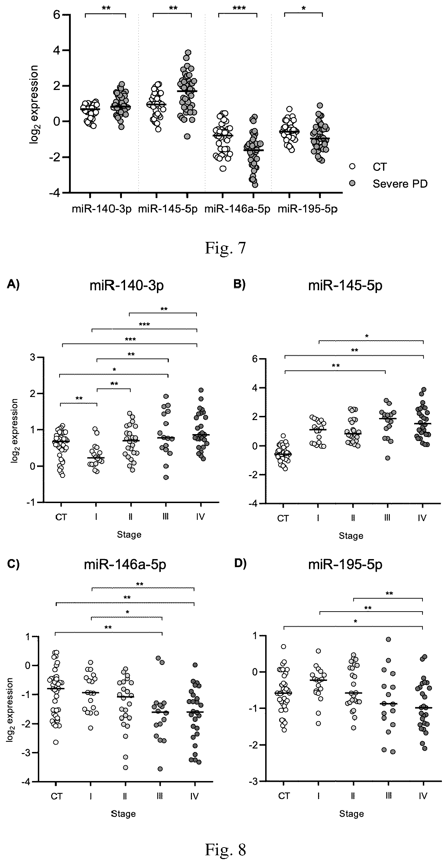

**Figure 7****.** Comparison of miR-140-3p, -145-5p, -146a-5p, and -195-5p level in gingival crevicular fluid collected from patients with severe periodontitis (PD) (Stage III and IV) and healthy controls (CT). The black lines denote median values, * P < 0.050, ** P < 0.010 and *** P < 0.001.
**Figure 8****.** Comparison of miR-140-3p, -145-5p, -146a-5p, and -195-5p level in gingival crevicular fluid collected from patients with different stages of periodontitis. The black lines denote median values, * P < 0.050, ** P < 0.010 and *** P < 0.001.

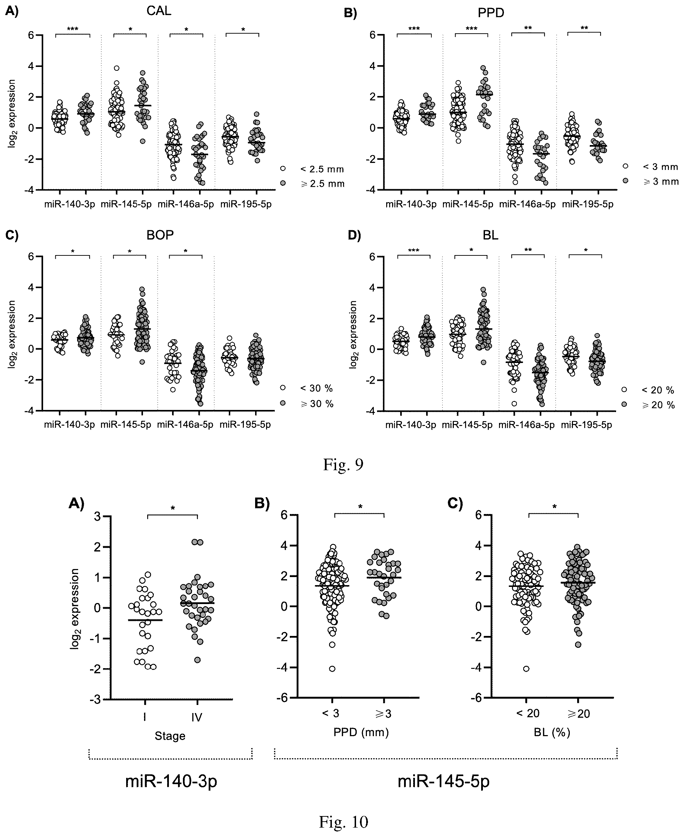

**Figure 9****.** Level of miR-140-3p, -145-5p, -146a-5p, and -195-5p in gingival crevicular fluid collected from periodontitis patients and controls and compared regarding the cut-off points of mean clinical attachment loss (CAL) (A), periodontal probing depth (PPD) (B), bleeding on probing (BOP) (C) and bone loss (BL) (D). The black lines denote median values, * P < 0.050, ** P < 0.010 and *** P < 0.001.
**Figure 10****.** Association between the level of salivary miRNAs and clinical-pathological periodontitis (PD) parameters: miR-140-3p with PD stage (A) and miR-145-5p with cut-off points of periodontal probing depth (PPD) (B) and bone loss (BL) (C). The black lines denote median values, * P < 0.050.

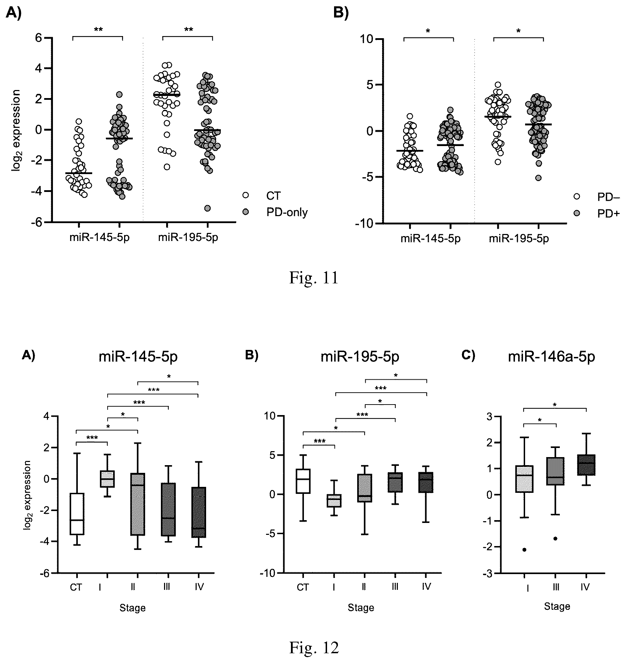

**Figure 11****.** Comparison of miR-145-5p and -195-5p level in blood plasma collected from patients with periodontitis (PD-only) *vs.* healthy controls (CT) (A) and all patients with PD (PD+) *vs.* periodontally healthy patients (PD-), regardless the status of rheumatoid arthritis (B). The black lines denote median values, * P < 0.050 and ** P < 0.010.
**Figure 12****.** Level of miR-145-5p (A), -195-5p (B), and 146a-5p (C) in blood plasma collected from patients with different stages of periodontitis (PD) and healthy controls (CT). The black lines inside the boxes indicate mean values, whiskers - data intervals, dots - outliers, * P < 0.050, ** P < 0.010 and *** P < 0.001.

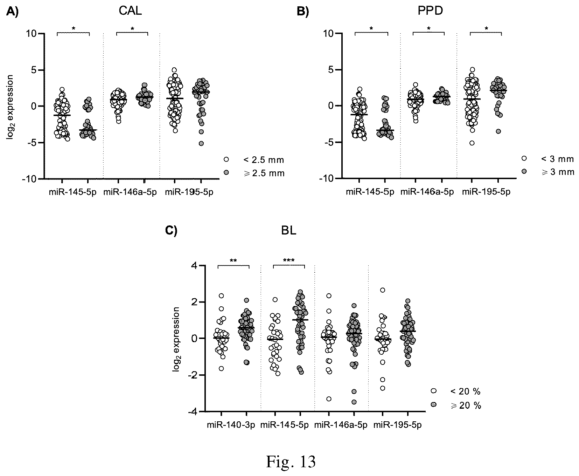

**Figure 13****.** Level of miR-145-5p, -146a-5p, and -195-5p in blood plasma collected from periodontitis patients and controls and compared regarding the cut-off points of mean clinical attachment loss (CAL) (A), periodontal probing depth (PPD) (B), and bone loss (BL) (C). The black lines denote median values, * P < 0.050 and ** P < 0.010.

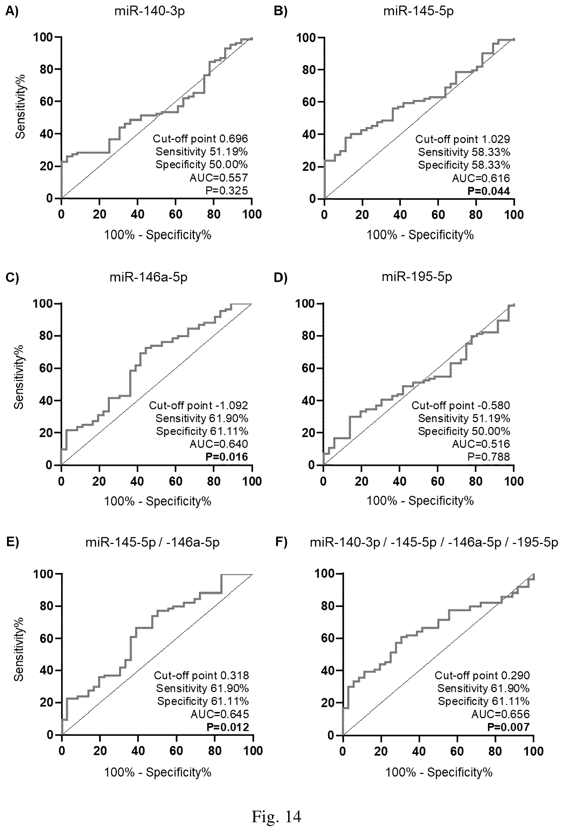

**Figure 14****.** ROC curve analysis of periodontitis (PD) biomarkers in gingival crevicular fluid (GCF): miR-140-3p (A), miR-145-5p (B), miR-146a-5p (C) and miR-195-5p (D), a combination of miR-145-5p / -146a-5p (E) and a combination of all miRNAs (F).

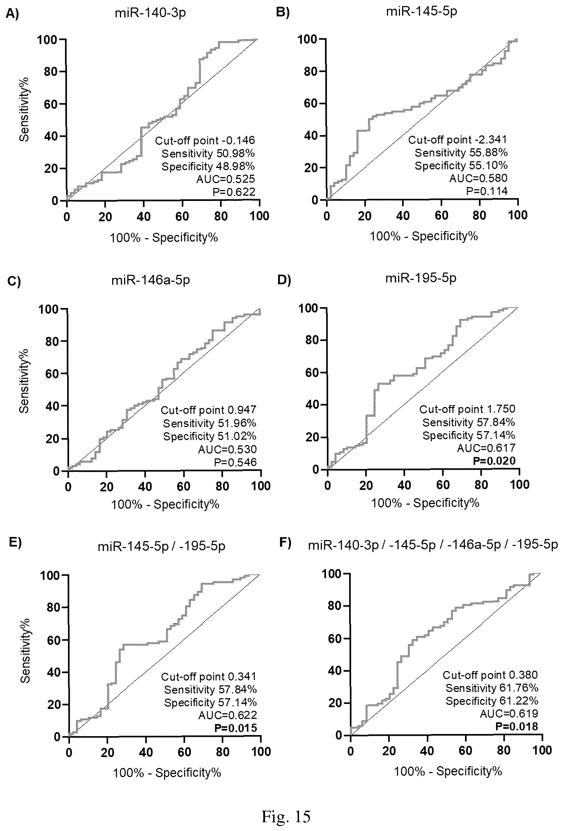

**Figure 15****.** ROC curve analysis of periodontitis (PD) biomarkers in plasma: miR-140-3p (A), miR-145-5p (B), miR-146a-5p (C) and miR-195-5p (D), a combination of miR-145-5p / -195-5p (E) and a combination of all miRNAs (F).

### DETAILED DESCRIPTION OF THE INVENTION

A study was designed with the aim of evaluating miRNA expression profiles in PD-affected and healthy gingival tissues while assessing the impact of autoimmune disease RA. Diagnostic utility of selected miRNAs was analyzed in gingival tissues, gingival crevicular fluid (GCF), saliva and blood plasma. A combination of miRNAs showing consistent expression profiles in different samples was selected for minimally- and non-invasive diagnostics of PD.

### 1. Participants

In total, 240 participants were enrolled in the study from Vilnius University Santaros and Zalgirio Clinics. Participants were categorized into two groups according to periodontal status:
1) PD+ group (Test) - 148 participants with a diagnosed PD, according to the 2018 American Academy of Periodontology (AAP) and the European Federation of Periodontology (EFP) classification of periodontal diseases guidelines were included in the study [17].
2) PD- group (Control) - 92 periodontally healthy participants.

A number of participants, who fulfilled the 2010 ACR/EULAR rheumatoid arthritis classification criteria [16] were invited to participate in the study. Sixty-four participants in PD+ group and 30 participants in PD- group were diagnosed with RA. All study groups are shown in Fig. 1.
Participants who had less than 8 teeth remaining, were pregnant, had diabetes mellitus or participants taking medication known to affect periodontal tissues (calcium channel blockers, antiepileptic drugs) were excluded from the research in order to eliminate any sources of bias.

The study was approved by Vilnius regional biomedical research ethics committee, approval No. 158200-18-922-500. A written informed consent was obtained from all participants before enrolment to the study. The research was carried out in accordance with the principles of the Declaration of Helsinki of 1975, revised in 2013.

### 2. Periodontal Examination

Clinical periodontal status assessment was performed by researcher periodontist performing full mouth probing at six-points and X-ray examination. The intra-examiner agreement of clinical assessment was calculated by means of Kappa coefficient and a value of 0.86 was obtained for clinical attachment loss (CAL) with difference of ±1 mm. The following measurements were recorded: 1) CAL, 2) probing pocket depth (PPD), 3) bleeding on probing (BOP), 4) alveolar bone loss (BL), 5) number of missing teeth. Third molars and dental implants were excluded. Based on these periodontal outcome parameters, patients were categorized using 2018 AAP / EFP definition of PD case and staging of periodontal disease [17, 18]. Accordingly, the presence of PD was defined as having PD-related detectable interdental CAL at ≥ 2 non-adjacent teeth or having a buccal or oral CAL ≥ 3 mm with pocketing > 3 mm detectable at ≥ 2 teeth. Patients were categorized into four stages: stage I - initial, stage II - moderate, stage III - severe, stage IV - advanced severe PD. Periodontal outcome parameters (namely PPD and BOP scores) were the primary outcome variables used to assess inflammatory activity in periodontal tissues.

Radiographic evaluation of mean BL value followed standardized protocol presented by Ryden and colleagues [19]. Distal and mesial sites of first and second molars were analyzed. Prior to examination, image quality of tooth sites was categorized in three groups (excellent, acceptable and unacceptable) according to modified California Dental Association (1977) guidelines [20]. Only sites with acceptable or excellent image quality as well as only teeth with visible crowns were assessed. The alveolar bone loss was determined as the proportion of the distance from cementoenamel junction to alveolar crest compared to root length.

A questionnaire was used to record anthropometric and sociodemographic variables including age, gender, body mass index (BMI), education, smoking status, alcohol consumption and oral health-related behaviours: frequency of tooth brushing, approximal tooth cleaning and annual dental prophylaxis.

### 3. Rheumatological evaluation

Clinical status of RA was assessed by an expert rheumatologist using disease activity score (DAS28), by counting 28 tender and swollen joints, visual analogue scale (VAS 100 mm), C-reactive protein concentration (CRP, mg/1) [21]. Disease duration as well as current RA treatment with glucocorticoids (Prednisolone, Methylprednisolone), synthetic disease-modifying antirheumatic drugs (sDMARDs), such as Methotrexate, Sulfasalazine, etc. and/or biologic disease-modifying anti-rheumatic drugs (bDMARDs) such as TNF-α inhibitors, IL-6 receptor antagonist, anti-CD20 monoclonal antibody, were recorded. Additionally RA impact of disease (RAID) and health assessment questionnaire (HAQ) were used to assess the impact of RA on patients' quality of life and disease itself [22, 23]. Seropositivity of RA (positive rheumatoid factor and/or anti-citrullinated protein antibodies (ACPA)) and CRP concentration were recorded from patients' medical data.

### 4. Sample Collection

Gingival samples were collected from all participants. Gingival tissues from patients with PD were obtained from the deepest periodontal pocket during periodontal treatment. Periodontally healthy participants' gingival tissue biopsies were taken during surgical crown lengthening or other interventional procedures performed for non-research-related purposes. Gingival tissue samples were stored in aqueous RNA stabilization solution RNAlater^{™} (Thermo Fisher Scientific (TFS), USA).

GCF sampling was performed following supragingival plaque and saliva removal and teeth isolation with cotton pellets. Sterile absorbent paper points were gently inserted into periodontal pocket and left in place for 30 seconds. For periodontally healthy participants, GCF collection was performed on palatal side of maxillary incisors. For one patient at least five paper points were inserted. Any points contaminated with blood were discarded. Paper points were pooled into 2 mL tube containing 20 µL aqueous RNA stabilization solution RNAlater^{™}.

Saliva sample collection was performed for all participants. Patients were asked to refrain from eating and drinking at least 2 h before saliva collection. Participants had to rinse their mouth with distilled water for 1 min and expectorate the water afterwards. Participants were then asked to spit at least 2 mL of saliva into a sterile tube. Following collection, saliva samples were centrifuged at 2600 × g for 15 min. Saliva supernatant was separated from cellular phase, transferred into 2 mL sterile tubes and RNase inhibitor SUPERase^{™} (TFS, USA) was added.

Blood plasma sample collection was performed for all participants. Venous whole blood samples were collected into sterile tubes containing dipotassium ethylenediaminetetraacetic acid (K2 EDTA) anticoagulant using 21-gauge needle into sterile BD Vacutainer^{®} tubes (Becton-Dickinson, Barricor^{™}, USA). Blood samples were centrifuged at 2000 × g for 15 min. Following centrifugation, plasma was transferred into 2 mL sterile tubes. All prepared samples were coded, labelled and stored at -80 °C.

### 5. RNA extraction

Snap-frozen gingival specimens were ground to a fine powder. Total RNA was isolated using Trizol^{™} (Invitrogen, TFS, USA) reagent according to the manufacturer's protocol and stored at - 80 °C. For RNA extraction from GCF, saliva and blood plasma miRNeasy Mini Kit (Qiagen) was used according to the manufacturer's instructions. QIAzol^{®} Lysis Reagent (Qiagen) was added to GCF, saliva and plasma samples and incubated for 5 min. Following incubation, 200 µL of chloroform was added and incubated for additional 2-3 min. As a positive control for extraction efficiency, 25 fmol cel-miR-39 was used. Extracted RNA was stored at -80 °C. The quality and quantity of RNA was determined using NanoDrop 2000 (Thermo Scientific, USA) spectrophotometer and Qubit 4 (Invitrogen) fluorometer.

### 6. MiRNA profiling by means of microarrays

For global miRNA profiling, a total of 16 samples were analyzed. Briefly, 100 ng of total RNA was spiked with control RNA Spike-In solution (Agilent Technologies (AT), USA), dephosphorylated, denatured and labeled with 3-pCp cyanine dye according to miRNA Microarray System with miRNA Complete Labeling and Hyb Kit (AT) protocol. After labeling, washed and dried samples were hybridized at 55 °C for 20 h on Human microRNA Microarray 8 x 60K arrays. Microarray images were acquired with Agilent SureScan scanner and analyzed using matching software *(Agilent Microarray Scan Control, Feature Extraction software v10.7, GeneSpring GX v14.9*) (AT, USA).

### 7. cDNA synthesis

For the expression analysis of selected miRNAs in **gingival tissue** biopsies RT reaction was performed by following manufacturer's protocol using *TaqMan*^{®} *MicroRNA Reverse Transcription Kit* and specific *RT Primer Pool* mix (Applied Biosystems (ABI), TFS). After 12 µL/1 reaction of RT reaction mix was assembled, vortexed and centrifuged, 3 µL (350 ng) of total RNA was added and incubated on ice for 5 min. The RT reaction was performed in a thermocycler at 16 °C for 30 min, 42 °C for 30 min, 85 °C for 5 min and was subsequently maintained at 4 °C.

Same *TaqMan*^{®} *MicroRNA Reverse Transcription Kit* was used for cDNA synthesis in **liquid biopsy** samples, but for every gene and every sample separate cDNA reactions were performed, differing in miRNA-specific stem-loop primers (*TaqMan*^{®} *MicroRNA Assay* for miR-140-3p, - 145, -146a, and -195; manufacturer's assay IDs - 002234, 002278, 000468 and 000494, respectively), following manufacturer's protocol (all from ABI, TFS) (Table 1). To 3.5 µL/1 reaction of reaction mix, 1.5 µL of miRNA assay and 2.5 µL (1-10 ng) of total RNA were added, centrifuged and then incubated in a thermocycler at the same conditions as described above. Synthesized cDNA was then either used immediately for the next step or stored at -20 °C.

**Table 1. Manufacturer's IDs and mature miRNA sequences of TaqMan^{®} Assays (Applied Biosystems, Thermo Fisher Scientific, USA).**

| **miRNA** | **ID** | **Mature miRNA Sequence** |
|---|---|---|
| miR-140-3p | 002234 | UACCACAGGGUAGAACCACGG |
| miR-145 | 002278 | GUCCAGUUUUCCCAGGAAUCCCU |
| miR-146a | 000468 | UGAGAACUGAAUUCCAUGGGUU |
| miR-195 | 000494 | UAGCAGCACAGAAAUAUUGGC |

### 8. Quantitative Reverse Transcription Polymerase Chain Reaction (RT-qPCR)

Expression level of selected miRNAs samples were quantified in triplicates using custom *TaqMan*^{®} *Low Density Arrays* in **gingival tissue** samples from patients with PD and/or RA and healthy controls by following manufacturer's instructions (ABI, TFS). Reaction mix, consisting of 2× *TaqMan Universal Master Mix II, No AmpErase UNG,* nuclease-free water and cDNA, was prepared for each sample separately. Array card was loaded by pipetting prepared reaction mix into the fill port of the channel. Cards were then centrifuged 2 times for 2 min at 2000 rpm, sealed and the fill reservoirs were trimmed off following the manufacturer's instructions. Array cards were run on the *ViiA7*^{™} *Real-Time PCR System* by using *ViiA 7 Software v1.2* (ABI, TFS) and the following temperature regime was applied: 2 min at 50 °C, 10 min at 95 °C, followed by 40 cycles of 15 s at 95 °C and 1 min at 60 °C.

miRNA abundance quantification in **liquid biopsy** samples was performed by RT-qPCR, which final reaction volume (20 µL/1 reaction) consisted of 2× *TaqMan*^{™} *Universal Master Mix II, no UNG, 20*× *TaqMan*^{™} *MicroRNA Assay* (both from ABI, TFS), 1.33 µL cDNA and RNase-free water and by following manufacturer's protocol. Each sample was run in triplicates and no-template control (NTC) was performed for evaluation of contamination. In NTC, nuclease-free water was added instead of the cDNA sample. When all samples were pipetted into the approproate wells, plate was sealed, briefly centrifuged to eliminate air bubbles and then loaded into the instrument. Reaction was performed on *ViiA7*^{™}*Real-Time PCR System* by using *ViiA* 7 *Software v1.2* and the following temperature regime was applied: 2 min at 50°C, 10 min at 95°C, followed by 50 cycles of 15 s at 95°C and 1 min at 60°C.

### 9. Statistical analysis

All data was analyzed using *SPSS* software, version 20.0 (IBM, Armonk, NY) and *GenEx* software, version 7.0 (MultiD Analyses AB, Sweden). Initially a Shapiro-Wilk normality tests was performed to check for variance homogeneity. miRNA data generated by RT-qPCR analysis was normalized using spiked-in cel-miR-39 and log-transformed (log2). Data was analyzed performing Student t-test and non-parametric tests. Associations were considered statistically significant when P ≤ 0.050 in all comparisons.

The diagnostic value for differentiating patients with PD and periodontally healthy participants was assessed by calculating the area under the receiver operating characteristic (ROC) curve (AUC). Associations were considered statistically significant when P ≤ 0.050 in all comparisons. ROC curves were generated using *GraphPad Prism v8.4.2* (GraphPad Software, Inc., San Diego, CA) and *MedCalc* (MedCalc Software bv, Ostend, Belgium) was used to perform logistic regression analysis for biomarker combinations.

### EXAMPLES

### Example 1. Selection and validation of miRNA combination for minimally- and non-invasive PD diagnostics

### Genome-wide miRNA expression profiling in gingival tissues

MiRNA expression profile was analysed in 16 gingival tissue specimens: PD-affected (PD+) (N = 8) and PD-unaffected (PD-) (N = 8) tissues. Out of 2569 available miRNAs, 760 were detected in ≥ 25% of samples, and only these were left in further analysis (Fig. 2A).

MiRNA expression analysis in gingival samples by means of microarrays revealed 177 differentially expressed miRNAs in PD-affected tissues compared to periodontally healthy gingival tissues (PD+ *vs.* PD-) (Fig. 2B). Majority of miRNAs (N = 140, 79.1%) were upregulated by >2-fold, including miR-30a-5p, -125a-3p, -126-5p, -1273g-3p, -140-3p, -145-5p, -146a-5p, - 155-5p, -195-5p, -575, -630, and -3917. Specificity for PD was evaluated by comparing patients with PD-only (PD+RA-, N = 4) vs. healthy controls (CT, N = 4) as 118 significantly deregulated miRNAs were revealed. Majority of miRNAs (N = 77, 65.3%) were upregulated (miR-140-3p, - 550a-3-5p, -765, -1273g-3p, -3917, etc.), while 41 (34.7%) miRNAs were downregulated (miR-1246, -210-3p, -375, etc.). To reveal which miRNAs may be associated with diseases of inflammatory nature, gingival samples from patients with PD and RA (PD+RA+, N = 4) *vs.* CT were compared. A general trend towards miRNA upregulation was observed as 22 out of 29 differentially expressed miRNAs were upregulated, including miR-550a-3-5p, -140-3p, and -765. Comparison of patients with RA-only (N = 4) and CT revealed 56 significantly deregulated miRNAs, of which 50 (89.3%) were downregulated (miR-34c-5p, -3620-5p, -330-5p, etc.), and 6 (10.7%) - upregulated (miR-339-3p, -711, -4701-5p, etc.).

Results of miRNA microarray analysis were used to select miRNAs related to PD in patients with or without RA. The selection of these miRNAs was based on fold changes and P-values as well as their possible role in inflammation or autoimmune processes. MiRNAs showing the most significant upregulation in PD+ *vs.* PD-, PD-only *vs.* CT, and PD+RA+ *vs.* CT were selected. Nine miRNAs (miR-30a-5p, -125a-3p, -145-5p, -146a-5p, -155-5p, -195-5p, -423-5p, -575, and -630) were significantly overexpressed in PD+ *vs.* PD- and PD+RA+ *vs.* CT groups, while 4 miRNAs (miR-126-5p, -195-5p, -1273g-3p, and -3917) were upregulated in PD+ *vs.* PD- and PD-only *vs.* CT. Significant overexpression of miR-140-3p and -765 was observed in all three of aforementioned groups and in PD-only *vs.* CT, respectively. Furthermore, both miR-140-3p and - 765 were significantly upregulated in PD+RA+ *vs.* CT group. All of the 15 selected miRNAs were downregulated in both of RA related groups (RA-only *vs.* CT and RA+ *vs.* RA-). However, only one significant association was identified in the case of miR-140-3p (FC = -1.8, P = 0.033) when patients with RA-only and CT were compared.

Based on revealed associations, 15 miRNAs were selected for further analysis, namely: miR-30a-5p, -125a-3p, -126-5p, -140-3p, -145-5p, -146a-5p, -155-5p, -195-5p, -423-5p, -550a-3-5p, - 575, -630, -765, -1273g-3p, and -3917. In order to validate detected changes, selected miRNAs were further analysed by performing RT-qPCR in an extended cohort of gingival tissues (N = 80).

### Selected miRNA expression analysis in gingival tissue

By performing RT-qPCR analysis of 80 gingival tissue samples, it was confirmed that a part of selected miRNAs was significantly associated with periodontitis. As well as in genome-wide miRNA expression analysis, overexpression of miR-145-5p and miR-140-3p (FC = 2.1, P < 0.0001 and FC = 1.4, P = 0.019, respectively) was identified in gingival tissue specimens from patients with PD-only (N = 25) *vs.* CT (N = 25) (Fig. 3). Comparison of gingival tissue specimens from PD+RA+ group (N = 23) and CT revealed that miR-140-3p was upregulated (FC = 1.5, P = 0.017) (Fig. 3), as well as in microarray analysis. Meanwhile, the expression of miR-125a-3p and miR-1273g-3p was significantly lower (FC < -1.5; P < 0.05) in PD+RA+ vs. CT and PD+ *vs.* PD- (N = 32 *vs.* 48). Analysis of miR-146a expression in gingival tissue yielded no significant results.

The expression of gingival miR-145-5p and miR-140-3p increased significantly amongst participants with severe PD (Stage III and IV). Comparison of severe PD *vs.* CT revealed that expression of miR-145 and miR-140-3p increased significantly amongst participants with PD (FC = 2.2, P < 0.001 and FC = 1.3, P = 0.026, respectively) (Fig. 4), while expression of miR-125a-3p decreased by 2.2-fold (P < 0.001). Significant difference in miR-145-5p gingival level was observed by comparing severe PD with moderate PD (Stage II) (FC = 2.0, P = 0.007). Comparison of moderate PD *vs.* CT revealed significantly higher expression of miR-195-5p (FC = 1.7, P = 0.038) and lower expression of miR-125a-3p (FC = -3.5, P = 0.013).

Also, significant associations of selected miRNAs were revealed with clinical-pathological parameters of PD, such as CAL, PPD, BOP and BL. In a group of patients with CAL score of ≥ 2.5 mm the expression of miR-145 was 2.2-fold higher (P < 0.001), while expression of miR-3917 - 1.6-fold lower (P = 0.022), as compared to the patients with CAL of < 2.5 mm (Fig. 5A). Upregulation of miR-145 (FC = 2.1, P < 0.001) in gingival tissue was also observed in participants with PPD of ≥ 3 mm as compared to the ones with PPD of < 3 mm (Fig. 5B). Overexpression of miR-145 (P < 0.001) and miR-140-3p (P = 0.009) as well as downregulation of miR-125a-3p by >2-fold (P = 0.001) was identified in patients with BOP of ≥ 33 % as compared to the ones with BOP of < 33 % (Fig. 5C). We also identified an upregulation of both miR-145 (P < 0.001) and miR-140-3p (P = 0.007) (Fig. 5D), as well as downregulation of miR-125a-3p (P = 0.001) when participants with BL of ≥ 20 % and BL of < 20 % were compared.

We identified no differentially expressed miRNAs between a group of RA-affected patients (RA-only) and healthy controls, however, comparison of RA patients (RA+) and rheumatologically healthy participants (RA-), regardless the status of PD, revealed downregulation of miR-1273g-3p (FC = -1.6, P < 0.001). Moreover, significant associations between miRNA expression and particular RA clinical variables were revealed. Comparison of patients with high RA activity (DAS28 of ≥ 5.1) and patients with DAS28-defined remission (DAS28 of < 2.6) indicated downregulation of miR-145 by 3.1-fold (P = 0.026). Also, RA patients who were not treated with bDMARDs had higher level of miR-140-3p (FC = 1.6, P = 0.004) as compared to participants receiving bDMARDs.

MiRNAs abundantly expressed in gingival tissues, namely, miR-140-3p, -145-5p, -146a-5p and -195, were selected for the analysis in liquid biopsy samples: GCF (N = 120), saliva (N = 173) and blood plasma (N =151). The selection of these miRNAs was based on fold changes, P-values, correlation between their expression and clinical-pathological parameters of PD, as well as their possible role in inflammation or autoimmune processes.

### Circulating miRNA analysis in bodily fluids

### 1. miRNA analysis in gingival crevicular fluid

GCF collected from patients with PD-only was related to lower level of miR-146a-5p (P = 0.044) as compared to CT (Fig. 6A). A lower level of miR-146a-5p and a higher level of miR-145-5p was also observed in PD+ *vs.* PD- (P < 0.001 and P = 0.027, respectively) (Fig. 6B). A higher level of miR-140-3p and a lower level of miR-195-5p was identified in both of aforementioned PD-affected groups as well, however, the differences were not statistically significant.

The abundance of miRNAs in GCF correlated with the severity of periodontal disease: an increased level of miR-140-3p (P = 0.001), miR-145 (P = 0.001) and a decreased level of miR-146a (P < 0.001), miR-195 (P = 0.042) was observed in GCF collected from patients with severe PD as compared to CT (Fig. 7).

Significant associations were found between GCF-derived miRNAs from different PD stages. Comparison of patients with Stage II PD and Stage I revealed a higher level of miR-140-3p (P = 0.002). Stage III PD group was associated with an increase in miR-140-3p level by 1.5-fold (P = 0.001) and a decrease in miR-146a amount by 1.7-fold (P = 0.010). Advanced severe (Stage IV) PD *vs.* initial (Stage I) PD comparison revealed that level of miR-140-3p (FC = 1.6, P < 0.001) and miR-145 (FC = 1.6, P = 0.030) was higher while level of miR-195 (FC = -1.5, P = 0.002) and miR-146a (FC = -1.7, P = 0.004) - lower for patients with Stage IV PD. Fig. 8 indicates changes in the level of miR-140-3p (A), -145-5p (B), -146a-5p (C), and -195-5p (D) regarding the different stages of PD.

Also, significant associations were identified between the level of GCF miRNAs and clinical variables of PD, such as CAL, PPD, BOP and BL. Patients with CAL score ≥ 2.5 mm had a higher level of miR-140-3p (P < 0.001), miR-145 (P = 0.025) and a lower level of miR-146a (P = 0.002) and miR-195 (P = 0.042) as compared to patients with CAL score < 2.5 mm (Fig. 9A). A group of patients with PPD ≥ 3 mm had a significantly higher level of miR-140 (FC = 1.4, P < 0.001) and miR-145 (FC = 1.8, P < 0.001) and a lower level of miR-195 (FC = -1.5, P <0.001) and miR-146a (FC = -1.7, P < 0.001) (Fig. 9B). Analysis regarding the BOP revealed a higher level of miR-140-3p (P = 0.012) as well as miR-145 (P = 0.026) and 1.4-fold lower level of miR-146a (P = 0.002) in a group of patients with BOP of ≥ 33 % (Fig. 9C). Analysis between two groups with different BL parameters revealed that the level of miR-140-3p (P < 0.001) and miR-145 (P = 0.001) was higher while miR-195 (P = 0.012) and miR-146a (P < 0.001) - lower for patients with BL of ≥ 20 % (Fig. 9D).

GCF collected from patients with RA-only was related to lower level of miR-140-3p (FC = - 1.3, P = 0.018) as compared to healthy participants. Same associations were also revealed with several RA clinical variables. Participants with high HAQ scores, indicating worse function and greater disability, had 1.5-fold lower level of miR-140-3p as compared to CT (P = 0.013). Moreover, a group of RA patients treated with bDMARDs had a significantly higher level of miR-146a (FC= 1.7, P = 0.023).

### 2. miRNA analysis in saliva

Analysis of selected miRNAs in saliva revealed a higher level of salivary miR-145-5p and - 195-5p and a lower level of -146a in samples collected from participants with both, PD-only and PD+RA+ groups, when compared to CT. Analogical differences were identified in PD+ *vs.* PD-comparison, however, the results were not statistically significant (P > 0.050). Significant difference in miRNA level was found between stages of PD: Stage IV PD was associated with 1.5-fold higher level of salivary miR-140 as compared to Stage I PD (P = 0.016) (Fig. 10A).

Significant associations were identified between the level of salivary miRNA and clinical-pathological parameters, such as PPD and BL. The level of circulating miR-145 was 1.5-fold higher in saliva from patients with both PPD of ≥ 3 mm and BL of ≥ 20 %, (Fig. 10B and 10C, respectively; P = 0.034 in both comparisons).

We also identified significant associations between salivary miRNAs level and HAQ score in RA patients. The level of circulating miR-140 was higher (FC = 1.7, P = 0.013) in medium and high scored patients. Moreover, a higher level of miR-195 (FC = 1.8, P = 0.017) was detected in saliva from patients with low, medium and high HAQ scores, compared to patients who are considered functionally normal according to the HAQ score.

### 3. MiRNA analysis in blood plasma

RT-qPCR analysis in blood plasma revealed that patients with PD-only were associated with a higher level of miR-145 (FC = 2.3, P = 0.002) and a lower level of miR-195 (FC = -2.6, P = 0.001) as compared to CT (Fig. 11A). Analogical differences were identified in PD+ vs. PD- cases (Fig. 11B).

It was identified that Stage I and Stage II PD was associated with a significant decrease in miR-195 (FC = -4.8, P < 0.001 and FC = -2.3, P = 0.013, respectively) and an increase in miR-145 plasma level (FC = 4.8, P < 0.001 and FC = 1.8, P = 0.037, respectively) as compared to CT. Significant associations were also found regarding the different stages of PD. Increased miR-195 (FC = 4.2, P < 0.001), -146a (FC = 1.6, P - 0.003) and decreased miR-145 (FC = -5.6, P < 0.001) plasma level was observed in Stage IV PD compared to Stage I PD. Analogical differences in the abundance of miR-195-5p (FC = 4.7, P < 0.001) and -145-4p (FC = -4.7, P < 0.001) were identified in Stage III PD *vs.* Stage I PD cases. In comparison to Stage III, Stage IV had 1.4-fold higher level of miR-146a in plasma (P = 0.016). Fig. 12 indicates changes in the level of miR-145-5p (A), -195-5p (B) and -146a-5p (C) in plasma samples from participants with different PD stages and CT.

Selected miRNAs showed different abundance in relation to PD clinical variables. In a group of patients with CAL score ≥ 2.5 mm, a significant increase in the level of miR-146a-5p and decrease in miR-145-5p was identified (P= 0.011 and P = 0.014, respectively) (Fig. 13A). Analysis regarding the PPD revealed a significantly higher level of plasma miR-195 (FC = 1.8, P = 0.039) and miR-146a (FC = 1.3, P = 0.024) and a lower level of miR-145 (FC = -2.2, P = 0.002) in a group of patients with PPD of ≥ 3 mm (Fig. 13B). Analogical differences were identified when associations with BL were analysed (Fig. 13C).

Analysis of selected miRNAs in plasma also revealed 2-fold increase in the level of miR-145-5p in RA cases, compared to healthy controls (P = 0.036). No significant associations were revealed between the level of selected miRNAs and clinical variables of RA.

### Diagnostic value of circulating miRNAs

To evaluate the diagnostic potential in distinguishing PD+ from PD- cases, ROC analysis was performed with selected miRNAs and AUC values were calculated for each miRNA and their combinations in GCF, saliva and plasma samples.

In GCF, miR-146a-5p demonstrated the highest AUC value of 0.640 (P = 0.016). Good diagnostic value was reached by measurement of GCF amount of miR-145-5p (AUC = 0.616, P = 0.044). Both sensitivity and specificity were higher in case of miR-146a, as compared to -145-5p (61.90 % vs. 58.33% and 61.11 % vs. 58.33 %, respectively). A combination of miR-145-5p/-146a-5p revealed an improved AUC value of 0.645 (P = 0.012) with similar 61.90 % sensitivity and 61.11 % specificity. Combining of all four selected miRNAs revealed a higher AUC value of 0.656 (P = 0.007) with similar sensitivity and specificity scores to that of aforementioned miR-146a-5p and combination of miR-145-5p/-146a-5p (Fig. 14).

In plasma, miR-195-5p alone demonstrated the highest AUC value of 0.617 (P = 0.020) with sensitivity and specificity of 57.84 % and 57.14 %, respectively. A combination of miR-145-5p/- 195-5p revealed an improved AUC value of 0.622 (P = 0.015) with similar sensitivity and specificity values to that of aforementioned miR-195-5p. Combining of all four selected miRNAs revealed slightly lower AUC value of 0.619 (P = 0.018), but higher sensitivity (61.76 %) and specificity (61.22 %) values (Fig. 15).

Meanwhile, analysis of salivary miRNAs yielded no significant AUC values.

### Example 2. Diagnostic kit suitable for the detection of inflammatory activity of periodontitis

A kit for the diagnosis of inflammatory activity of periodontitis was assembled, comprising:
a) sterile tubes and paper points for GCF sample collection and RNA stabilization solution; sterile tubes containing anticoagulant for venous whole blood sample collection; sterile tubes and RNase inhibitors for saliva sample collection and salivary RNA stabilization;
b) silica-membrane based materials for RNA extraction from the biological fluid samples;
c) primers, enzymes and buffers for cDNA synthesis;
d) primers, hydrolysis probes and master mix for inflammatory miRNA quantification.

### CONCLUSION

We evaluated miRNA expression profiles in PD-affected and healthy gingival tissues collected from patients with and without RA. Thorough validation enabled us to distinguish four miRNAs (miR-140-3p, -145-5p, -146a-5p and -195-5p) that were significantly deregulated in PD-affected tissues as compared to healthy gingival tissues. Aforementioned miRNAs are known as key regulators of the immune system and inflammatory processes and play an important role in PD-associated processes such as periodontal tissue destruction and osteoclastogenesis. Analysis of abundance of selected miRNAs in liquid biopsy (GCF, saliva and plasma) samples indicated that the level of selected miRNAs significantly differed between PD-affected and healthy individuals and the higher level of these inflammatory-related miRNAs correlated with worse clinical-pathological parameters of PD. Moreover, the assessment of diagnostic properties of miR-140-3p, -145-5p, -146a-5p, and -195-5p revealed that combination of these four miRNAs can be used as biomarkers for minimally- and non-invasive diagnostics of PD in patients with and without RA. Optimal cut-off points of relative level of combined miRNAs in GCF and plasma samples are - 0.290 (Fig. 14F) and 0.380 (Fig. 15F), respectively._Furthermore, it enables assessment of PD activity that is relevant to the choice of further periodontal treatment tactics. It should be noted that, for those skilled in the art, some variations and modifications can be made without departing from the scope of the present invention and therefore, the protection scope of the invention shall be subject to the appended claims.

### References:

1. R. C. Page, P. I. Eke. Case definitions for use in population-based surveillance of periodontitis. J Periodontol. 2007;78:1387-99.
2. P. I. Eke, B.A. Dye, L. Wei, G. O. Thornton-Evans, and R.J. Genco. Prevalence of Periodontitis in Adults in the United States: 2009 and 2010. J DENT RES 2012 Oct;91(10):914-20
3. Tonetti MS, Jepsen S, Jin L, Otomo-Corgel J. Impact of the global burden of periodontal diseases on health, nutrition and wellbeing of mankind: A call for global action. J Clin Periodontol. 2017;44(5):456-462. doi:10.1111/jcpe.12732
4. Bansal M, Khatri M, Taneja V. Potential role of periodontal infection in respiratory diseases - a review. J Med Life. 2013;6(3):244-248.
5. Sanz M, Marco Del Castillo A, Jepsen S, et al. Periodontitis and cardiovascular diseases: Consensus report. J Clin Periodontol. 2020;47(3):268-288. doi:10.1111/jcpe.13189
6. Dhotre S V, Davane M S, Nagoba B S. Periodontitis, Bacteremia and Infective Endocarditis: A Review Study, Arch Pediatr Infect Dis. 2017 ; 5(3):e41067. doi: 10.5812/pedinfect.41067.
7. Chapple IL. Time to take periodontitis seriously. BMJ 2014;348:g2645.
8. C. Xiu-Min, H. Qing-Chun, Y. Sheng-Li, C. Yong-Liang, Y. Yu-Hong, H. Ling, H. Yu, H. Run-Yue, PhD. Role of Micro RNAs in the Pathogenesis of Rheumatoid Arthritis Medicine (Baltimore). 2015 Aug; 94(31): 1326
9. Hussain SB, Botelho J, Machado V, et al. Is there a bidirectional association between rheumatoid arthritis and periodontitis? A systematic review and meta-analysis. Semin Arthritis Rheum. 2020;50(3):414-422. doi:10.1016/j.semarthrit.2020.01.009
10. Cosgarea R, Tristiu R, Dumitru RB, et al. Effects of non-surgical periodontal therapy on periodontal laboratory and clinical data as well as on disease activity in patients with rheumatoid arthritis. Clin Oral Investig. 2019;23(1):141-151. doi:10.1007/s00784-018-2420-3
11. K. Ishida, T. Kobayashi, S. Ito, Y. Komatsu, T. Yokoyama, M. Okada, et al. Interleukin-6 gene promoter methylation in rheumatoid arthritis and chronic periodontitis. J Periodontol. 2012;83(7):917-25.
12. I. Silosi, M. Cojocaru, L. Foia, M. V. Boldeanu, F. Petrescu, P. Surlin, V. Biciusca. Significance of Circulating and Crevicular Matrix Metalloproteinase-9 in Rheumatoid Arthritis-Chronic Periodontitis Association. Journal of Immunology Research. 2015; 218060 - 6.
13. S. O. Geerts, V. Legrand, J. Charpentier, A. Albert, E. H. Rompen. Further evidence of the association between periodontal conditions and coronary artery disease. J Periodontol 2004;75:1274-80.
14. Lang NP, Bartold PM. Periodontal health. J Clin Periodontol. 2018;45 Suppl 20:S9-S16. doi:10.1111/jcpe.12936
15. Saresella M, La Rosa F, Piancone F, et al. The NLRP3 and NLRP1 inflammasomes are activated in Alzheimer's disease. Mol Neurodegener. 2016;11:23. Published 2016 Mar 3. doi:10.1186/s13024-016-0088-1
16. Liu T, Zhang L, Joo D, Sun SC. NF-κB signaling in inflammation. Signal Transduct Target Ther. 2017;2:17023-. doi:10.1038/sigtrans.2017.23
17. Gaber T, Strehl C, Buttgereit F. Metabolic regulation of inflammation. Nat Rev Rheumatol. 2017;13(5):267-279. doi:10.1038/nrrheum.2017.37
18. R. Dai, S. A. Ahmed. MicroRNA, a new paradigm for understanding immunoregulation, inflammation, and autoimmune diseases. Transl Res 2011; 157:163-179.
19. Fushimi S, Nohno T, Nagatsuka H, Katsuyama H. Involvement of miR-140-3p in Wnt3a and TGFβ3 signaling pathways during osteoblast differentiation in MC3T3-E1 cells. Genes Cells. 2018;23:517-27
20. Taguchi YH, Murakami Y. Principal component analysis based feature extraction approach to identify circulating microRNA biomarkers. PLoS One. 2013;8(6):e66714
21. O'Leary L, Sevinç K, Papazoglou IM, Tildy B, Detillieux K, Halayko AJ, et al. Airway smooth muscle inflammation is regulated by microRNA-145 in COPD. FEBS Lett (2016) 590:1324-34. doi:10.1002/1873-3468.12168
22. Yang B, Kang X, Xing Y, Dou C, Kang F, Li J, et al. Effect of microRNA-145 on IL-1β-induced cartilage degradation in human chondrocytes. FEBS Lett (2014) 588:2344-52. doi:10.1016/j.febslet.2014.05.033
23. He M, Wu N, Leong MC, et al. miR-145 improves metabolic inflammatory disease through multiple pathways. J Mol Cell Biol. 2020;12(2):152-162. doi:10.1093/jmcb/mjz015
24. Kutty RK, Nagineni CN, Samuel W, Vijayasarathy C, Jaworski C, Duncan T, et al. Differential regulation of microRNA-146a and microRNA-146b-5p in human retinal pigment epithelial cells by interleukin-1β, tumor necrosis factor-a, and interferon-y. Mol Vis (2013) 19:737-50.
25. Xu W-D, Lu M-M, Pan H-F, Ye D-Q. Association of MicroRNA-146a with autoimmune diseases. Inflammation (2012) 35(4):1525-9. doi:10.1007/s10753-012-9467-0
26. Haumik D, Scott G, Schokrpur S, Patil C, Campisi J, Benz C. Expression of microRNA-146 suppresses NF-κB activity with reduction of metastatic potential in breast cancer cells. Oncogene (2008) 27:5643-7. doi:10.1038/onc.2008.171
27. Zheng C, Shu Y, Luo Y, Luo J. The role of miR-146a in modulating TRAF6-induced inflammation during lupus nephritis. Eur Rev Med Pharmacol Sci (2017) 21:1041-8.
28. Li M, Wang J, Fang Y, Gong S, Li M, Wu M, et al. MicroRNA-146a promotes mycobacterial survival in macrophages through suppressing nitric oxide production. Sci Rep (2016) 6:23351. doi:10.1038/srep23351
29. Caton J.G., Armitage G., Berglundh T., Chapple I.L.C., Jepsen S., Kornman K.S., Mealey B.L., Papapanou P.N., Sanz M., Tonetti M.S. A new classification scheme for periodontal and peri-implant diseases and conditions -Introduction and key changes from the 1999 classification. J. Clin. Periodontol. 2018;45:S1-S8. doi: 10.1111/jcpe.12935
30. Ji S, Choi Y. Point-of-care diagnosis of periodontitis using saliva: technically feasible but still a challenge. Front Cell Infect Microbiol. 2015;5:65. Published 2015 Sep 3. doi:10.3389/fcimb.2015.00065
31. Bonneau E, Neveu B, Kostantin E, Tsongalis GJ, De Guire V. How close are miRNAs from clinical practice? A perspective on the diagnostic and therapeutic market. EJIFCC. 2019;30(2):114-127. Published 2019 Jun 24.
32. Armitage GC. Development of a classification system for periodontal diseases and conditions. Ann Periodontol. 1999; 4: 1- 6.

## Claims

1. A method for diagnosing inflammatory activity of periodontitis in biological fluid samples of patients with and without rheumatoid arthritis comprising the steps of:
a. sample collection by using at least five sterile absorbent paper points that are inserted into periodontal pocket and left in place for a up to a minute to collect GCF and by pooling said paper points into sterile tubes containing aqueous RNA stabilization solution; by collecting at least 2 mL of saliva into a sterile tubes from instructed patients, spinning down saliva samples at 2600 × g for 15 min before transferring supernatant to sterile tubes and adding RNase inhibitor for stabilization; by collecting venous whole blood samples into sterile tubes containing anticoagulant and by centrifuging collected whole blood samples at 2000 × g for 15 min before transferring plasma to sterile tubes;
b. performing RNA isolation from said samples using an RNA extraction procedure;
c. performing targeted cDNA synthesis to reverse transcribe extracted miRNAs;
d. performing RT-qPCR to detect inflammatory miRNAs in said samples;
e. analyzing the data to determine the level of combined inflammatory miRNAs related to PD.

2. The method according to claim 1, wherein the anticoagulant used is dipotassium ethylenediaminetetraacetic acid.

3. The method according to claim 1, wherein the RNA stabilization solution stabilizes RNA in unfrozen, intact samples and is compatible with subsequent RNA isolation methods.

4. The method according to claim 1, wherein RNase inhibitor is non-human origin and protein-based, noncovalently binding and inhibiting multiple RNases.

5. The method according to claim 1, wherein said RNA extraction procedure comprises a phenol/guanidine-based lysis of samples with silica-membrane based purification of total high-purity RNA, including <200 nt length RNAs, suitable for all downstream applications.

6. A kit for the diagnosis of inflammatory activity of periodontitis, comprising:
a. sterile tubes and paper points for GCF sample collection and RNA stabilization solution; sterile tubes containing anticoagulant for venous whole blood sample collection; sterile tubes and RNase inhibitors for saliva sample collection and salivary RNA stabilization;
b. silica-membrane based materials for RNA extraction from the biological fluid samples;
c. primers, enzymes and buffers for cDNA synthesis;
d. primers, hydrolysis probes and master mix for inflammatory miRNA quantification.

7. The method according to claim 1 and the kit according to claim 8, wherein the biological fluids are gingival crevicular fluid, saliva and blood plasma.

8. The method according to claim 1 and the kit according to claim 8, wherein the inflammatory miRNAs are: miR-140-3p, miR-145-5p, miR-146a-5p and miR-195-5p.
